Europäisches Patentamt

**European Patent Office**

Office européen des brevets

(19)

(11) Publication number : **0 387 933 B1**

(12)

# EUROPEAN PATENT SPECIFICATION

(45) Date of publication of patent specification :
04.08.93 Bulletin 93/31

(51) Int. Cl.⁵ : **A23L 1/308, A21D 2/16**

(21) Application number : **90200485.2**

(22) Date of filing : **01.03.90**

(54) **High fiber baked cookies containing psyllium.**

(30) Priority : **16.03.89 US 324773**
**08.09.89 US 404722**

(43) Date of publication of application :
**19.09.90 Bulletin 90/38**

(45) Publication of the grant of the patent :
**04.08.93 Bulletin 93/31**

(84) Designated Contracting States :
**AT BE CH DE DK ES FR GB GR IT LI LU NL SE**

(56) References cited :
**EP-A- 0 144 644**
**EP-A- 0 309 029**
**US-A- 4 619 831**
**PATENT ABSTRACTS OF JAPAN, vol. 11, no.**
**141(C-421)(2588), 8 May 1987; & JP-A-61282046**
**(Haruo Kajitani) 12.12.1986**

(73) Proprietor : **THE PROCTER & GAMBLE**
**COMPANY**
**One Procter & Gamble Plaza**
**Cincinnati Ohio 45202 (US)**

(72) Inventor : **Cappel, James Wilbur**
**4837 Potomac Dr.**
**Fairfield, OH 45014 (US)**
Inventor : **Courts, Julia Marie**
**3853 Hyde Park Avenue No. 2**
**Cincinnati, OH 45209 (US)**
Inventor : **Prosise, Robert Lawrence**
**7104 Larchwood Dr.**
**Cincinnati, OH 45241 (US)**

(74) Representative : **Suslic, Lydia et al**
**Procter & Gamble European Technical Center**
**N.V. Temselaan 100**
**B-1853 Strombeek-Bever (BE)**

EP 0 387 933 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

BACKGROUND OF THE INVENTION

The present invention relates to novel high fiber baked cookies, and to methods for preparing these cookies. These cookies essentially comprise psyllium and insoluble dietary fiber, flour, shortening and water at levels within a specified range. These baked cookies have excellent texture, mouthfeel and palatability. They are useful as dietary aids in the control of bowel function (including use as laxatives) and/or for reducing blood cholesterol levels and/or for weight control or other indications where fiber may be beneficial.

Generally speaking, compositions containing psyllium plus other fiber materials are not new. U.S. Patent 4,784,861, to Gori, issued November 15, 1988, describes powders formed of a mixture of oat, wheat and corn bran mixed with pectin, guar gum, psyllium and cutin to which mineral supplements have been added. U.S. Patent 4,619,831, to Sharma, issued October 28, 1986, describes dietary fiber products comprising insoluble dietary fiber (92-98.5%) coated or enrobed with soluble dietary fiber (1.5-8%; psyllium is mentioned as one of many soluble fibers). U.S. Patent 4,565,702, to Morley et al., issued January 21, 1986, describes dietary fiber compositions comprising dietary fibers which are insoluble fibers coated with soluble fiber. U.S. Patent 4,348,379, to Kowalsky et al., issued September 7, 1982, describes dietetic compositions comprising psyllium seed, linseed, and wheat bran. European Patent Application Publication No. 144,644, published June 19, 1985 by G. D. Searle and Co., describes high fiber food compositions comprising psyllium and other dietary fiber sources.

EP-A-0 309 029 describes cookies containing psyllium or psyllium and polyol polyesters.

West German Patent Specification 2,430,509, published January 15, 1976 by Hypolab S.A., Genf. (Schweiz), describes preparing compositions containing bulk laxatives (including psyllium mucilloid) in the form of a cake. The cake dough is prepared and baked in molds to produce cakes having thickness of 3-6 mm.

Reduced calorie baked cookies containing microcrystalline cellulose as a preferred bulking agent are disclosed in U.S. Patent 4,668,519, to Dartey et al., issued May 26, 1987. This patent indicates that these cookies can optionally include bulking agents such as dietary fibers (including psyllium fiber) at levels up to about 10% by weight of the dough.

Great Britain Patent Specification 1,590,507, published June 3, 1981, by Syntex (U.S.A.) Inc., describes compositions comprising mixtures of purified cellulose and pectin as a source of dietary fiber. The effectiveness of these compositions for controlling fecal output in humans is compared versus various other compositions, including biscuits which comprise only psyllium.

Several other U.S. patents describe non-baked compositions in which psyllium is described as an optional or essential ingredient: 4,778,676, to Yang et al., issued October 18, 1988 (describes chewable compositions comprising a precoated active and a confectionery matrix); 4,766,004, to Moskowitz, issued August 23, 1988 (describes dietary fiber supplement compositions comprising whole psyllium husks having a particle size of from 12 to 70 mesh, food grade vegetable fat which is a solid at room temperature, sweetening agent and flavoring agent); 4,737,364, to Kalogris, issued April 12, 1988 (describes low calorie dry food concentrate); 4,698,232, to Sheu et al., issued October 6, 1987 (describes fiber-containing confectionery compositions comprising dietary fiber pretreated with a lubricant, a foamed matrix, and an amorphous matrix); and 4,551,331, to Rudin, issued November 5, 1985 and R.E. 32,811, issued December 27, 1988 (describe dietary fiber products comprising a dietary fiber coated with a food grade emulsifier).

In addition to the preceding publications, the following publications are also worth mentioning herein: U.S. Patent 3,219,455, to Dubois, issued November 23, 1965; U.S. Patent 4,461,782, to Robbins et al., issued July 24, 1984; U.S. Patent 3,954,976, to Mattson et al., issued May 4, 1976; U.S. Patent 4,005,195, to Jandacek, issued January 25, 1977; U.S. Patent 4,005,196, to Jandacek et al., issued January 25, 1977; U.S. Patent 4,034,083, to Mattson, issued July 5, 1977; U.S. Patent 3,455,714, to Bishop et al., issued July 15, 1969; U.S. Patent 4,321,263, to Powell et al., issued March 23, 1982; U.S. Patent 4,673,578, to Becker et al., issued June 16, 1987; U.S. Patent 4,568,557, to Becker et al., issued February 4, 1986; U.S. Patent 2,060,336, to Near et al., issued April 5, 1933; U.S. Patent 4,156,021, to Richardson, issued May 22, 1979; U.S. Patent 4,089,981, to Richardson, issued May 16, 1978; U.S. Patent 3,023,104, to Battista, issued February 27, 1962; U.S. Patent 4,511,561, to Madaus et al., issued April 16, 1985; U.S. Patent 4,459,280, to Colliopoulos et al., issued July 10, 1984; U.S. Patent 4,341,805, to Chaudhary, issued July 27, 1982; U.S. Patent 4,181,747, to Kickle et al., issued January 1, 1980; U.S. Patent 4,350,714, to Duvall, issued September 21, 1982; U.S. Patent 4,315,954, to Kuipers, issued February 16, 1982; U.S. Patent 3,992,147, to Christian et al., issued November 16, 1976; U.S. Patent 3,148,114, to Fahrenbach et al., issued September 8, 1964; U.S. Patent 4,639,367, to Mackles, issued January 27, 1987; French Patent Specification 2,165,795, published December 31, 1971; European Patent Application Publication No. 105,195, published April 11, 1984; European Patent Application Publication No. 285,201, published October 5, 1988; Great Britain Patent Specification 2,067,402, published July 30, 1981; Great Britain Patent Specification 2,201,875, published September 14, 1988; World Patent Application Publication No. 80/00658, published April 17, 1980; Goodman & Gilman, The Pharmacologic Basis of Therapeutics, Fifth Edition, 979 (1975); Garvin et al., Proc. Soc. Exp. Biol. Med., 120, 744-746 (1965); Forman et al., Proc. Soc. Exp. Biol. Med., 127, 1060-1063 (1968); Anderson et al., Fed. Proc., 46, 877 (1987); Anderson et al., Am. J. Gastroenterol., 81, 907-919 (1986); and Faberberg, Curr. Ther. Res., 31, 166 (1982).

In spite of the large amount of research aimed at developing portable and palatable compositions containing psyllium, there is a continuing need to provide additional compositions having psyllium at levels high enough to provide therapeutic benefits in reasonably sized, portable compositions having good eating aesthetics. It has been discovered that such compositions can be prepared in the form of a cookie by using insoluble fiber with psyllium fiber in compositions having a certain level of shortening. By using the insoluble fiber in these compositions, it has been surprisingly discovered that the eating quality of the cookie compositions are greatly enhanced, especially with regard to the stickiness of the composition (i.e., the tendency of the composition to stick to and/or form an unpleasant coating on the teeth during ingestion), while allowing for the use of shortening at levels lower than would otherwise be necessary to avoid undesirable aesthetics. Such compositions, and the methods for making them, are also well suited for large scale manufacture of portable, palatable and efficacious psyllium-containing cookies.

It is an object of the present invention to provide compositions which are convenient, portable and highly palatable (e.g., having excellent texture and mouthfeel) psyllium-containing cookies. An object of the present invention is also to provide convenient, portable psyllium-containing compositions having good consumer acceptance to promote compliance with a regimen for providing laxation benefits and/or reducing serum cholesterol levels and/or weight control. A further object is to provide psyllium-containing compositions having little or no gummy or rubbery texture and reduced stickiness during ingestion. Also, an object is to provide portable psyllium-containing compositions which are efficacious for providing laxation benefits and/or reducing serum cholesterol levels. An object of the present invention is also to provide methods for preparing these efficacious baked cookie compositions.

These and other objects of the present invention will become readily apparent from the detailed description which follows.

All percentages and ratios used herein are by weight unless otherwise specified, and all measurements are made at 25°C unless otherwise specified.

## SUMMARY OF THE INVENTION

The present invention relates to highly palatable psyllium-containing baked cookie compositions. These compositions essentially comprise: from about 10% to about 20% psyllium fiber, from about 5% to about 17% of an insoluble dietary fiber, from about 13% to about 20% of a shortening component, from about 10% to about 40% of a flour component, from about 5% to about 40% of a sugar component, and from about 1.5% to about 3.5% water.

The present invention also relates to methods for making the baked cookie compositions of the present invention. These methods comprise the steps of: (a) mixing to a uniform mass the psyllium fiber (and preferably the insoluble fiber) with a saturated aqueous solution of sugar components; (b) mixing a shortening component with the mass following step (a); (c) mixing with the mass of step (b), either sequentially or all at once, the remaining components of the cookie dough; and (d) baking the dough to reduce the water content of the cookie composition to within from about 1.5% to about 3.5% by weight of the baked cookie composition.

The present invention further relates to methods for reducing serum cholesterol levels, and for providing laxation and regulating bowel function. These methods comprise orally administering to a human in need of such treatment a safe and effective amount of a baked cookie composition of the present invention.

## DETAILED DESCRIPTION OF THE INVENTION

### Baked Cookie Compositions Containing Psyllium:

The present invention relates to high fiber psyllium-containing baked cookie compositions. These compositions comprise: (a) psyllium fiber; (b) insoluble dietary fiber; (c) shortening component; (d) flour component; (e) sugar component; and (f) water. The essential and optional components for use in compositions of the present invention, and the amounts to be utilized, are described in detail hereinafter.

(a) Psyllium Fiber:

The present cookie compositions comprise psyllium fiber. The term "psyllium fiber", as used herein, means the seed coat of psyllium seed (either intact or macerated or otherwise comminuted).

Psyllium fiber comes from psyllium seed, from plants of the Plantago genus. Various species such as Plantago lanceolate, P. rugelii, and P. major, are known. Commercial psyllium includes the French (black; Plantago indica), Spanish (P. psyllium) and Indian (blonde; P. ovata). Indian (blonde) psyllium is preferred for use herein.

Intact or macerated seeds can be used in the practice of this invention. However, it is typical to remove the seed coats from the rest of the seed by, for example, slight mechanical pressure, and then to use only the seed coat. In the practice of the present invention it is convenient and desirable to use macerated seed coat in the final composition. The seed coat is therefore preferably removed and sanitized by methods known in the art (e.g., ethylene oxide) prior to use in the present composition. Furthermore, the psyllium fiber utilized preferably has high purity, being about 85% to about 100% pure, and more preferably being about 95% to about 100% pure.

The baked cookie compositions of the present invention essentially comprise from about 10% to about 20% psyllium fiber, preferably from about 11% to about 18% psyllium fiber, and more preferably from about 12% to about 15% psyllium fiber, by weight of the baked composition.

(b) Insoluble Dietary Fiber:

The present cookies also comprise insoluble dietary fiber. The term "insoluble dietary fiber", as used herein, means the water insoluble, substantially non-swellable component of fiber material safe for human ingestion which is non-digestible and non-metabolizable by humans.

A wide range of materials containing insoluble dietary fiber may be used in the present invention. Preferred are cereal brans and mixtures thereof, due to their relatively high content of insoluble dietary fiber. Also preferred is that these cereal brans comprise at least about 75% of the insoluble dietary fiber. Cereal brans useful in the present invention include those selected from the group consisting of wheat, corn, barley, rye, oats and mixtures thereof. Most preferred are oat or corn. The components of the insoluble dietary fiber from these cereal brans are known to be cellulose, hemicellulose and lignin.

When the insoluble dietary fiber content of a composition of the present invention is to be determined, an analytical technique which may be used is disclosed in the Association of Analytical Chemist publication "Total Dietary Fiber: AOAC Collaborative Study", January 25, 1982,. This technique utilizes enzymatic and chemical procedures to isolate the dietary fiber. When wheat bran or corn bran for example is treated according to this AOAC method, the recoverable dietary fiber is an insoluble fiber. The bran is first treated with a solvent, e.g., petroleum ether or hexane, to remove the fat. The defatted bran is then digested enzymatically with protease. Finally, the bran is treated with alpha or beta-amylase and amyloglucosidase. The recoverable material is protein-free, fat-free and carbohydrate-free insoluble dietary fiber. The insoluble dietary fiber content of a variety of dietary fiber sources are also disclosed in publications, including for example "Plant Fiber in Foods" by James W. Anderson, M.D. (published by the HCF Diabetes Research Foundation, Inc., Lexington, Ky.; 1986).

The baked cookie compositions of the present invention essentially comprise from about 5% to about 17% of an insoluble dietary fiber, preferably from about 5% to about 10% insoluble dietary fiber, and more preferably from about 7% to about 9% insoluble dietary fiber, by weight of the baked compositions.

(c) Shortening Component:

The present cookie compositions further comprise a shortening component. Fats which can be used as the shortening component can be any of the usual fat stocks employed in preparing liquid, fluid, plastic, or solid shortenings, preferably having a solid content of less than about 25 at room temperature, more preferably having a solid content of less than about 10 at room temperature, and most preferably having a solid content of about 0 at room temperature. Various fats such as cottonseed oil, soybean oil, lard, and other vegetable, animal and marine fats, or mixtures thereof, either unhydrogenated or in various stages of hydrogenation, can be used. Suitable shortening components can also be formulated with non-absorbable, non-digestible fatty acid esters of polyols, in particular sucrose polyesters (disclosed in U.S. Patent 4,005,196 to Jandacek et al., issued January 25, 1977.), and/or other non-nutritive or reduced calorie fat substitute materials suitable for use in the present compositions.

The baked cookie compositions of the present invention essentially comprise from about 13% to about 20% of the shortening component, preferably from about 15% to about 19% shortening component, and more preferably from about 16% to about 18% shortening component, by weight of the baked cookie composition.

### (d) Flour Component:

The present cookie compositions also comprise a flour component. Any type of flour which is suitable in cookie doughs can be used in the present invention. For example, suitable flours include wheat flour, rye flour, corn flour, cottonseed meal, and sorghum flour. preferably, wheat flour is used in preparing the cookie compositions for the present invention. This flour can be bleached or unbleached. Furthermore, starches may constitute a portion of the flour component of the present compositions (preferably less than about 5% of the baked composition).

Most preferred are cookie compositions comprising pregelatinized food starch (e.g., pregelatinized wheat starch; pregelatinized corn starch). Examples of such starches include: Sta-Mist 7415 starch, Sta-Mist 463 starch, and Sta-Mist 454 starch (all sold by A.E. Staley Manufacturing Company; Decatur, Illinois). The baked cookie compositions preferably comprise from about 1% to about 4%, and more preferably from about 1.5% to about 3%, of pregelatinized food starch by weight of the baked cookie composition.

The baked cookie compositions of the present invention comprise from about 10% to about 40% flour component, preferably from about 15% to about 30% flour component, and more preferably from about 17% to about 23% flour component, by weight of the baked cookie compositions.

### (e) Sugar Component:

The present compositions also comprise a sugar component. Suitable sugar components include sucrose, invert sugar syrups, brown sugar, corn syrup solids, fructose, dextrose (glucose), honey, molasses and maple syrup. Particularly preferred sugar components are sucrose, fructose and corn syrup solids.

The baked cookie compositions of the present invention comprise from about 5% to about 40% of a sugar component, preferably from about 20% to about 40% of a sugar component, and more preferably from about 25% to about 35% of a sugar component, by weight of the baked compositions.

### (f) Water:

The cookie compositions of the present invention also comprise water in the range of from about 1.5% to about 3.5%, preferably from about 2% to about 3%, and more preferably about 2.0% to about 2.5%, by weight of the baked composition. Thus, while the dough prior to baking contains substantially more water than this, the dough is baked for a time and at a temperature sufficient to reduce the water content in the present cookie compositions to this level.

The water content of the cookie dough prior to baking is typically in the range of from about 5% to about 15% by weight of the dough. It is to be noted that the weight percentages of the essential components hereinbefore stated are by weight of the cookie composition following baking. The weight percentages of these essential components in the dough are therefore proportionally reduced by an amount which depends on the level of water present in the dough. Finally, it is preferred that the cookie dough be baked soon after preparation since storage of the dough could adversely effect the efficacy and/or aesthetics of the cookie.

### (g) Optional Components:

The cookie compositions of the present invention may further optionally comprise other components compatible with the psyllium and other essential cookie components, and which are suitable for ingestion. In particular, such components must not significantly reduce the efficacy of the psyllium for the therapeutic uses described herein (especially laxation and/or cholesterol reduction).

One such preferred optional component is one or more emulsifiers, frequently referred to as "dough conditioners" because they are used to control the consistency of the dough. Suitable optional emulsifiers include mono- and diglycerides and fatty acids, sucrose partial fatty acid esters, sorbitan esters of fatty acids, polyoxyethylene sorbitan esters of fatty acids, propylene glycol esters, polyethylene glycol esters, ethoxylated mono- and diglycerides, fumarated esters of monoglycerides or their alkali metal salts, alkanoyl lactylates or their metal salts and lecithins,. Specific dough conditioners include sorbitan monostearate (Span 60), polyoxyethylene sorbitan monostearate (Tween 60), propylene glycol monostearate, glycerol lactopalmitate, sodium stearoyl fumarate, calcium stearoyl-2-lactylate, ethoxylated monoglycerides and lecithin. The amount of emulsifier can be varied to obtain the dough properties desired.

Another preferred optional component is one or more leavening agents. Non-yeast leavening agents include a source of carbon dioxide such as sodium bicarbonate or potassium bicarbonate, alone or in combination with a leavening acid such as monocalcium phosphate, dicalcium phosphate sodium acid pyrophosphate, sodium aluminum sulfate, sodium aluminum phosphate, potassium acid tartrate and the like. The amount of leavening agent used depends on the particular agent employed and the leavening characteristics desired.

Other optional components which may be included in the dough are milk products such as whole milk, skim milk, buttermilk, whey, concentrated milk product (condensed or evaporated milk), dried milk products, nonfat milk powder, dry whole milk, modified whole milk, egg products, including egg whites and egg yolks, protein sources (e.g., soy protein), non-nutritive artificial sweeteners (e.g., aspartame, acesulfame, saccharin, cyclamate), spices, cocoa powder, flavors such as vanilla, salt, color additives, preservatives and antioxidants. Furthermore, other pharmaceutical active agents may be included as desired into the composition. Such actives include, for example, sennosides (which are laxatives; preferably from about 0.04% to about 0.25% by weight of the baked cookie composition), analgesics, cholesterol reduction agents.

Method for Making the Present Cookie Compositions:

While the dough to be baked to form the present cookie compositions may be prepared in any manner which does not substantially reduce the efficacy of the psyllium for the therapeutic uses described herein (especially laxation and/or cholesterol reduction), the present invention further relates to a method for preparing the present cookie compositions. This method comprises the steps of: (a) mixing to a uniform mass the psyllium fiber (and preferably the insoluble dietary fiber) with a saturated aqueous solution of sugar components; (b) mixing a shortening component with the mass following step (a); (c) mixing with the mass following step (b), either sequentially or all at once, the remaining components of the cookie dough; and (d) baking the dough to reduce the water content of the cookie composition to within from about 1.5% to about 3.5% by weight of the baked cookie composition.

More preferred is the method comprising the steps of: (a) mixing to a uniform mass the psyllium fiber and insoluble dietary fiber with a saturated aqueous solution of sugar components, wherein said saturated sugar solution comprises from about 12% to about 28% of water by weight of the saturated sugar solution; (b) mixing a shortening component with the mass following step (a); (c) mixing the flour component with the mass following step (b); and (d) baking the dough to reduce the water content to within from about 1.5% to about 3.5% by weight of the baked cookie composition.

Temperature and time conditions generally suitable for baking cookie compositions can be used in preparing the baked cookie compositions of the present invention. Typically, the cookie dough is baked at a temperature of from about 325° to about 450°F (from about 162° to about 232°C), for from about 9 to about 5 minutes (depending on the baking temperature). Preferably, the cookie dough is baked at a temperature of from about 325° to about 360°F (from about 162° to about 182°C), for from about 8 to about 7 minutes. The particular baking conditions employed depend upon the size of the cookie dough being baked, the particular level of water desired in the final product, the particular oven used, and like factors. The term "baking", as used herein, means radiant, conductive, or convective exposure to energy of a type which imparts thermal energy to the cookie dough being baked. It thus includes conventional, convection, dielectric and microwave oven baking.

The compositions of the present invention can be used in order to provide laxation and regulating bowel function for a human in need of such treatment. Ingestion of from about 2.5 grams to about 30 grams per day of the psyllium fiber in a cookie composition according to the present invention is appropriate in most circumstances to produce laxation.

However, this can vary with the size and condition of the patient, and such matters will, of course, be apparent to the attending physician. However, since the psyllium material is nontoxic, even higher ingestion levels can be used without undue side effects. A typical dose for laxation purposes involves administering from about 3 to about 15 grams of psyllium fiber in one dose.

The compositions of the present invention can be used to reduce serum cholesterol levels in humans. Ingestion of compositions of the present invention comprising amounts sufficient to administer from about 2.5 grams to about 30 grams per day of psyllium fiber, preferably from about 5 grams to about 15 grams, is appropriate in most circumstances. However, this can vary with the size and condition of the patient, and the patient's blood cholesterol level. Such matters will, of course, be apparent to the attending physician. However, since the psyllium material is nontoxic, even higher ingestion levels can be used without undue side effects, keeping in mind the materials herein have the hereinbefore noted laxative effect.

Treatment of the patient to reduce serum cholesterol levels comprises chronic ingestion in order to lower and maintain the lowered cholesterol levels. Daily ingestion is preferred, and a daily ingestion of from about 5 grams to about 15 grams of the psyllium fiber is most commonly used, with said ingestion preferably being at 2 or 3 regularly spaced intervals throughout the day. Again, depending on the patient's size and cholesterol level in the patient's blood, this can be varied.

Reducing serum cholesterol levels in humans comprises chronic ingestion in order to lower and maintain the lowered cholesterol levels. Daily ingestion is preferred, and a daily ingestion of from about 5 grams to about 15 grams of the psyllium fiber is most commonly used, with said ingestion preferably being at 2 or 3 regularly spaced intervals throughout the day. Again, depending on the patient's size and cholesterol level in the patient's blood, this can be varied.

EXAMPLE I

Oatmeal Cookies

A psyllium-containing cookie composition according to the present invention is prepared having the following components:

| Component | Wet Weight % (by weight of dough) |
|---|---|
| Fructose | 5.00 |
| Sucrose | 20.28 |
| Molasses | 0.50 |
| DRO Water a) | 7.60 |
| Psyllium b) | 12.45 |
| Oat Fiber c) | 6.50 |
| Crisco® Oil d) | 15.00 |
| Soy Lecithin | 1.00 |
| DRO Water | 1.42 |
| Sodium Bicarbonate | 0.20 |
| Flour e) | 19.50 |
| Rolled Oats f) | 9.00 |
| Vanilla Flavor | 0.20 |
| Butter Flavor | 0.20 |
| Cinnamon | 0.90 |
| Nutmeg | 0.25 |
| | 100.00 |

a) DRO water = double reverse osmosis purified water
b) Ethylene oxide sanitized, sized psyllium mucilloid
c) 80% microfine oat hull fiber sold by Canadian Harvest, St. Thomas, Ontario, Canada; approximately 80% insoluble fiber
d) Sold by The Procter & Gamble Company, Cincinnati, Ohio
e) Unbleached, undried cookie flour sold by Acme Evans, Indianapolis, Indiana
f) About 6.2% insoluble dietary fiber

The manufacturing procedure is as follows:
1. Add fructose, sucrose, molasses and DRO water to the mixer and mix 2 minutes (Sweet Dough Mixer sold by Peerless Machinery Corporation, Sidney, Ohio; at 22 rpm);
2. Add psyllium and oat fiber to the mixer and mix 2 minutes;

3. Add crisco oil and lecithin to the mixer and mix 2 minutes;

4. Add dro water, soda, cinnamon, nutmeg, vanilla and butter flavor to the mixer and mix for 1 minute;

5. Add flour to the mixer and mix 2 minutes;

6. Add oats and mix for 1 minute, scrape down and mix another minute;

7. Empty finished dough batch into trough;

8. Empty trough into rotary dough hopper and rotary mould rectangles having correct dimensions (approx. 6.35cm length X 4.1cm width X 0.76cm height);

9. Bake in a 3 zone oven (Wernaire Automatic Recirculating Oven; Werner Lahara, Grand Rapids, Michigan) for 7-8 minutes (approximate zone temperature between 325-360°F (162 to 182°C); dictated by quality);

10. Allow to cool on a wire mesh belt for approx. 10 minutes.

These baked cookies contain the following amounts of essential ingredients: 12.8% psyllium fiber; 8% insoluble dietary fiber; 17.2% shortening component; 19.4% flour component; 28.9% sugar component; and 2.2% water.

Consumption of 2 of these cookies daily by a person in need of laxation provides effective laxative benefits and regulates bowel function. These cookies are convenient to use and provide psyllium in a very palatable form.

Substitution of about one-third of the Crisco® Oil component in this cookie composition with a non-absorbable, non-digestible sucrose polyester (a mixture of sucrose hexa-, hepta-, and octaesters of soybean oil fatty acids) provides a reduced calorie cookie composition according to the present invention. These cookies are also particularly well suited for use in reducing serum cholesterol levels in humans in need of such benefit. Ingestion of 6 of these cookies, taken in portions of 2 cookies at three regularly spaced intervals throughout the day, is desirable for this purpose.

EXAMPLE II

Chocolate Cookie

A psyllium-containing cookie composition according to the present invention is prepared having the following components:

| Component | Wet Weight % (by weight of dough) |
|---|---|
| Fructose | 7.50 |
| Sucrose | 22.16 |
| Water | 4.77 |
| Psyllium | 12.45 |
| Oat Fiber | 6.49 |
| Crisco® Oil | 15.00 |
| Soy Lecithin | 1.00 |
| Water | 4.83 |
| Soda | 0.10 |
| Custard Flavor | 0.05 |
| Chocolate Flavor | 0.40 |
| Cocoa | 5.50 |
| Flour | 19.75 |
| | 100.00 |

The manufacturing procedure is as follows:

1. Add fructose, sucrose and water to the mixer and mix 2 minutes (at 22 rpm);

2. Add psyllium and oat fiber to the mixer and mix 2 minutes;

3. Add crisco oil and lecithin to the mixer and mix 2 minutes;

4. Add water, soda, custard flavor, and chocolate flavor to the mixer and mix 1 minute;

5. Add cocoa and flour to the mixer and mix 1 minute, scrape down and mix another minute;

6. Empty finished dough batch into trough;

7. Empty trough into rotary dough hopper and rotary mould rectangles having correct dimensions (approx. 6.35cm length X 4.1cm width X 0.76cm height).

8. Bake in a 3 zone oven for 7-8 minutes (approximate zone temperature between 325-360°F (162° to 182°C); dictated by quality);

9. Allow to cool on a wire mesh belt for approx. 10 minutes.

These baked cookies contain the following amounts of essential ingredients: 12.7% psyllium fiber; 7% insoluble dietary fiber; 17.7% shortening component; 19.5% flour component; 33.7% sugar component; and 2.3% water.

Consumption of 2 of these cookies daily by a person in need of laxation provides effective laxative benefits and regulates bowel function. These cookies are convenient to use and provide psyllium in a very palatable form.

EXAMPLE III

Senna/Psyllium Cookie

A cookie composition according to the present invention containing psyllium and senna is prepared (by a manufacturing procedure similar to that described in Example I) having the following components:

| Component | Wet Weight %<br>(by weight of dough) |
|---|---|
| Fructose | 5.00 |
| Sucrose | 20.45 |
| Molasses | 0.50 |
| Water | 7.60 |
| Psyllium | 12.45 |
| Crisco® Oil | 15.00 |
| Soy Lecithin | 1.00 |
| Water | 1.42 |
| Sodium Bicarbonate | 0.20 |
| Vanilla Flavor | 0.20 |
| Butter Flavor | 0.20 |
| Oats | 9.00 |
| Flour | 19.50 |
| Cinnamon | 0.90 |
| Senna a) | 0.08 |
| Oat Fiber | 6.50 |

a) 60% calcium sennosides supplied by P. L. Thomas & Co., Inc., Bernardsville, New Jersey.

These baked cookies contain about the following amounts of essential ingredients: 12.8% psyllium fiber; 8% insoluble dietary fiber; 17.2% shortening component; 19.4% flour component; 28.8% sugar component;

and 2.2% water.

Consumption of 2 of these cookies daily by a person in need of laxation provides effective laxative benefits.

EXAMPLE IV

Oatmeal Cookies

A psyllium containing cookie composition according to the present invention is prepared having the following components:

| Component | Wet Weight % (by weight of dough) |
|---|---|
| Fructose | 6.94 |
| Sucrose | 17.69 |
| Molasses | 0.50 |
| Water | 6.44 |
| Psyllium | 12.68 |
| Oat Fiber | 6.55 |
| Corn Oil | 15.10 |
| Lecithin | 1.00 |
| Starch[a] | 2.00 |
| Water | 1.43 |
| Soda | 0.20 |
| Cinnamon | 0.91 |
| Nutmeg | 0.25 |
| Vanilla Flavor | 0.20 |
| Butter Flavor | 0.20 |
| Ascorbic Acid | 0.15 |
| Flour | 18.71 |
| Oats | 9.05 |

[a] Sta-Mist 7415 starch supplied by A.E. Staley Manufacturing Company, Decatur, Illinois.

The manufacturing procedure is as follows:

1. Add fructose, sucrose, molasses and water to the mixer and mix 1 minute (at 22 rpm);
2. Add psyllium and oat fiber to the mixer and mix 2 minutes;
3. Add corn oil and lecithin to the mixer and mix 2 minutes;
4. Add starch to the mixer and mix for 2 minutes;
5. Add water, soda, cinnamon, nutmeg, vanilla, butter flavor and ascorbic acid to the mixer and mix for 1 minute;
6. Add flour to the mixer and mix 2 minutes;
7. Add oats and mix for 1 minute, scrape down and mix another minute;
8. Empty finished dough batch into trough;
9. Empty trough into rotary dough hopper and rotary mould rectangles having correct dimensions (approx. 6.35cm length X 4.1cm width X 0.76cm height);
10. Bake in a 3 zone oven for 7-8 minutes (approximate zone temperature between 325-395°F (162 to 202°C); dictated by quality);

11. Allow to cool on a wire mesh belt for approx. 10 minutes and package appropriately.

Consumption of 2 of these cookies daily by a person in need of laxation provides effective laxative benefits and regulates bowel function. These cookies are convenient to use and provide psyllium in a very palatable form.

## Claims

1. Psyllium-containing baked cookie compositions comprising:
   (a) from 10% to 20% psyllium fiber;
   (b) from 5% to 17% of an insoluble dietary fiber;
   (c) from 13% to 20% of a shortening component;
   (d) from 10% to 40% of a flour component:
   (e) from 5% to 40% of a sugar component; and
   (f) from 1.5% to 3.5% water.

2. Psyllium-containing baked cookie compositions comprising:
   (a) from 11% to 18% psyllium fiber;
   (b) from 5% to 10% of an insoluble dietary fiber;
   (c) from 15% to 19% of a shortening component;
   (d) from 15% to 30% of a flour component;
   (e) from 20% to 40% of a sugar component; and
   (f) from 2% to 3% water.

3. Psyllium-containing baked cookie compositions comprising:
   (a) from 12% to 15% psyllium fiber;
   (b) from 7% to 9% of an insoluble dietary fiber;
   (c) from 16% to 18% of a shortening component;
   (d) from 17% to 23% of a flour component;
   (e) from 25% to 35% of a sugar component; and
   (f) from 2% to 2.5% water.

4. Psyllium-containing baked cookie compositions according to any of Claims 1-3 wherein the insoluble dietary fiber comprises one or more cereal brans.

5. Psyllium-containing baked cookie compositions according to any of Claims 1-4 wherein the insoluble dietary fiber comprises cereal bran selected from the group consisting of wheat, corn, barley, rye, oats and mixtures thereof.

6. Psyllium-containing baked cookie compositions according to any of Claims 1-5 further comprising from 1% to 4% of pregelatinized food starch.

7. Psyllium-containing baked cookie compositions according to any of Claims 1-6 wherein the shortening component comprises a non-absorbable, non-digestible fatty acid ester of polyols.

8. Psyllium-containing baked cookie compositions according to any of Claims 1-7 further comprising sennoside.

## Patentansprüche

1. Psyllium enthaltende, gebackene Kekszusammensetzungen, welche enthalten:
   (a) 10 % bis 20 % Psylliumfasern;
   (b) 5 % bis 17 % an unlöslichen Diätfasern;
   (c) 13 % bis 20 % einer Backfettkomponente;
   (d) 10 % bis 40 % einer Mehlkomponente;
   (e) 5 % bis 40 % einer Zuckerkomponente; und
   (f) 1,5% bis 3,5% Wasser.

**2.** Psyllium enthaltende, gebackene Kekszusammensetzungen, welche enthalten:
(a) 11 % bis 18 % Psylliumfasern;
(b) 5 % bis 10 % an unlöslichen Diätfasern;
(c) 15 % bis 19 % einer Backfettkomponente;
(d) 15 % bis 30 % einer Mehlkomponente;
(e) 20 % bis 40 % einer Zuckerkomponente; und
(f) 2 % bis 3 % Wasser.

**3.** Psyllium enthaltende, gebackene Kekszusammensetzungen, welche enthalten:
(a) 12 % bis 15 % Psylliumfasern;
(b) 7 % bis 9 % an unlöslichen Diätfasern;
(c) 16 % bis 18 % einer Backfettkomponente;
(d) 17 % bis 23 % einer Mehlkomponente;
(e) 25 % bis 35 % einer Zuckerkomponente; und
(f) 2 % bis 2,5 % Wasser.

**4.** Psyllium enthaltende, gebackene Kekszusammensetzungen nach einem der Ansprüche 1 bis 3, worin die unlöslichen Diätfasern eine oder mehrere Getreidekleien umfassen.

**5.** Psyllium enthaltende, gebackene Kekszusammensetzungen nach einem der Ansprüche 1 bis 4, worin die unlöslichen Diätfasern eine Getreidekleie umfassen, welche aus einer Gruppe ausgewählt ist, die aus Weizen, Mais, Gerste, Roggen, Hafer und Gemischen hievon besteht.

**6.** Psyllium enthaltende, gebackene Kekszusammensetzungen nach einem der Ansprüche 1 bis 5, welche weiterhin 1 % bis 4 % einer vorgelatinierten Nahrungsmittelstärke enthalten.

**7.** Psyllium enthaltende, gebackene Kekszusammensetzungen nach einem der Ansprüche 1 bis 6, worin die Backfettkomponente einen nicht resorbierbaren, unverdaulichen Fettsäureester von Polyolen umfaßt.

**8.** Psyllium enthaltende, gebackene Kekszusammensetzungen nach einem der Ansprüche 1 bis 7, welche weiterhin Sennoside enthalten.


**Revendications**

**1.** Compositions de galettes cuites contenant du psyllium comprenant :
(a) 10 % à 20 % de fibres de psyllium,
(b) 5 % à 17 % d'une fibre diététique insoluble,
(c) 13 % à 20 % d'un composant de type graisse,
(d) 10 % à 40 % d'un composant de type farine,
(e) 5 % à 40 % d'un composant de type sucre, et
(f) 1,5 % à 3,5 % d'eau.

**2.** Compositions de galettes cuites contenant du psyllium comprenant :
(a) 11 % à 18 % de fibres de psyllium,
(b) 5 % à 10 % d'une fibre diététique insoluble,
(c) 15 % à 19 % d'un composant de type graisse,
(d) 15 % à 30 % d'un composant de type farine,
(e) 20 % à 40 % d'un composant de type sucre, et
(f) 2 % à 3 % d'eau.

**3.** Compositions de galettes cuites contenant du psyllium comprenant :
(a) 12 % à 15 % de fibres de psyllium,
(b) 7 % à 9 % d'une fibre diététique insoluble,
(c) 16 % à 18 % d'un composant de type graisse,
(d) 17 % à 23 % d'un composant de type farine,
(e) 25 % à 35 % d'un composant de type sucre, et
(f) 2 % à 2,5 % d'eau.

4.  Compositions de galettes cuites contenant du psyllium selon l'une quelconque des revendications 1 à 3, dans lesquelles la fibre diététique insoluble comprend un ou plusieurs sons de céréales.

5.  Compositions de galettes cuites contenant du psyllium selon l'une quelconque des revendications 1 à 4, dans lesquelles la fibre diététique insoluble comprend un son de céréale choisi dans le groupe formé par le blé, le maïs, l'orge, le seigle, l'avoine et leurs mélanges.

6.  Compositions de galettes cuites contenant du psyllium selon l'une quelconque des revendications 1 à 5 comprenant par ailleurs 1 % à 4 % d'amidon alimentaire pré-gélatinisé.

7.  Compositions de galettes cuites contenant du psyllium selon l'une quelconque des revendications 1 à 6, dans lesquelles le composant de type graisse comprend un ester d'acides gras et de polyols non absorbable et non digestible.

8.  Compositions de galettes cuites contenant du psyllium selon l'une quelconque des revendications 1 à 7 comprenant par ailleurs un sennoside.